(19) Europäisches Patentamt
European Patent Office
Office européen des brevets

(11) Veröffentlichungsnummer: **0 256 982 B1**

# (12) EUROPÄISCHE PATENTSCHRIFT

(45) Veröffentlichungstag der Patentschrift: **28.08.91**

(51) Int. Cl.5: **C07B 53/00**, C07C 209/00, B01J 31/24

(21) Anmeldenummer: **87810435.5**

(22) Anmeldetag: **29.07.87**

## (54) Verfahren zur Herstellung von optisch aktiven sekundären Arylaminen.

(30) Priorität: **04.08.86 CH 3116/86**

(43) Veröffentlichungstag der Anmeldung:
**24.02.88 Patentblatt 88/08**

(45) Bekanntmachung des Hinweises auf die Patenterteilung:
**28.08.91 Patentblatt 91/35**

(84) Benannte Vertragsstaaten:
**AT BE CH DE ES FR GB IT LI NL SE**

(56) Entgegenhaltungen:
**EP-A- 0 000 403**
**EP-A- 0 031 877**
**EP-A- 0 104 375**

**Fieser & Fieser, Reagents for Organic Synthesis, vol. 9, John Wiley & Sons, New York, NY (US)**

**CHEMICAL ABSTRACTS, Band 94, Nr. 5, 2. Februar 1981, Columbus, Ohio, USA, Seite 494, Spalte 2, Abstrakt Nr. 29871m; M.I. KALINKIN et al.: "Catalytic ionic hydrogenation of azomethines"; & DOKL: ADAD. NAUK SSSR 1980, 253(5), 1137-9**

(73) Patentinhaber: **CIBA-GEIGY AG**
**Klybeckstrasse 141**
**CH-4002 Basel(CH)**

(72) Erfinder: **Spindler, Felix, Dr.**
**Dullikerstr. 392**
**CH-4656 Starrkirch-Wil(CH)**
Erfinder: **Pugin, Benoit, Dr.**
**Wasserhausweg 16**
**CH-4142 Münchenstein(CH)**

EP 0 256 982 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von optisch aktiven sekundären Aminen durch asymmetrische Hydrierung von prochiralen N-Arylketiminen mit chiralen Iridiumdiphosphin- oder -diphosphinitkomplexen.

In der EP-A-0 104 375 sind chirale 2,2'-Diphosphin-diphenyl-liganden beschrieben, deren Komplexe mit Metallen der Gruppe VIII des Periodensystems, bevorzugt mit Rhodium, als Katalysatoren bei der asymmetrischen Hydrierung von α-(Acylamin)-acrylsäuren verwendet werden können. Fieser and Fieser's Reagents for Organic Synthesis, Vol. 9, S. 259-260 beschreiben die gleiche Umsetzung mit anderen cyclischen Diphosphinliganden und Rhodiumkomplexen. Diese Publikation beruht auf der EP-A-0 000 403. In der EP-A-0 031 877 sind chirale 1,2-(Diphenylphosphin)alkane und deren Metallkomplexe mit Platinmetallen, bevorzugt Rhodiumkomplexen, als homogene Katalysatoren für die asymmetrische Hydrierung von α-(Acylamin)-acrylsäuren beschrieben.

Es wurde gefunden,dass Iridiumverbindungen mit chiralen Diphosphinoder Diphosphinitliganden geeignete homogene asymmetrische Katalysatoren für die Hydrierung von prochiralen N-Arylketiminen sind. Diese Reaktion führt bei hohen chemischen Umsätzen und guten optischen Ausbeuten zu optisch aktiven sekundären N-Arylaminen. Optisch aktiv bedeutet einen Ueberschuss eines Enantiomeren mit R- oder S-Konfiguration.

Gegenstand vorliegender Erfindung ist ein Verfahren zur Herstellung von optisch aktiven sekundären N-Arylaminen der Formel I

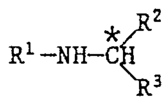

(I),

worin $R^1$ $C_6$-$C_{12}$-Aryl oder über ein Ring-C-Atom gebundenes $C_4$-$C_{11}$-Heteroaryl mit 1 oder 2 Heteroatomen im Ring bedeutet, die durch $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CM, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, -CONR$^4$ R$^5$ oder -COOR$^4$ substituiert sein können, Sekundäramino mit 2 bis 24 C-Atomen,

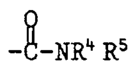

oder -COOR$^4$, wobei $R^4$ und $R^5$ unabhängig voneinander $C_1$-$C_{12}$-Alkyl, Phenyl oder Benzyl oder $R^4$ und $R^5$ zusammen Tetra- oder Pentamethylen oder 3-Oxapentylen sind, substituiert sein können;

$R^2$ und $R^3$ voneinander verschieden sind und gegebenenfalls durch -OH, -CN, Halogen, $C_1$-$C_{12}$-Alkoxy, Phenoxy, Benzyloxy, Sekundäramino mit 2 bis 24 C-Atomen,

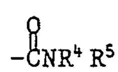

oder -COOR$^4$ substituiertes $C_1$-$C_{12}$-Alkyl oder Cycloalkyl mit 3-8 Ring-C-Atomen, gegebenenfalls wie $R^1$ substituiertes $C_6$-$C_{12}$-Aryl oder $C_7$-$C_{16}$-Aralkyl, -CONR$^4$R$^5$ oder -COOR$^4$ darstellen, worin $R^4$ und $R^5$ die zuvor angegebene Bedeutung haben; oder

$R^1$ die zuvor angegebene Bedeutung hat und $R^2$ und $R^3$ zusammen gegebenenfalls durch I oder 2 -O-, -S- oder -NR$^4$- unterbrochenes, und/oder gegebenenfalls durch =O oder wie zuvor für $R^2$ und $R^3$ in der Bedeutung von Alkyl substituiertes, und/oder mit Benzol, Furan, Thiophen oder Pyrrol kondensiertes Alkylen mit 2 bis 5 C-Atomen sind,

und * für überwiegend R- oder S-Konfiguration steht, durch asymmetrisch katalysierte Hydrierung von N-arylierten prochiralen Ketiminen der Formel II

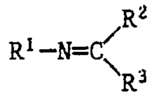 (II),

worin $R^1$, $R^2$ und $R^3$ die zuvor angegebene Bedeutungen haben, in Gegenwart von Komplexsalzen eines Edelmetalls mit chiralen Liganden, das dadurch gekennzeichnet ist, dass man die Hydrierung bei einer Temperatur von -20 bis 80 °C und einem Wasserstoffdruck von $10^5$ Pa bis $6 \bullet 10^6$ Pa vornimmt und dem Reaktionsgemisch katalytische Mengen einer Iridiumverbindung der Formel III oder IIIa

[XIrYZ] (III) oder $[XIrY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Ir-Atom einen 5-, 6- oder 7-Ring bildet, oder Y ein chirales Diphosphinit, dessen Phosphinitgruppen über 2 C-Atome verknüpft sind und das zusammen mit dem Ir-Atom einen 7-Ring bildet, bedeutet, Z für Cl, Br oder J steht, und $A^{\ominus}$ das Anion einer Sauerstoff- oder Komplexsäure darstellt.

$R^1$ kann in beliebigen Stellungen durch gleiche oder verschiedene Reste substituiert sein, z.B. mit 1 bis 5, vorzugsweise 1 bis 3 Substituenten. Die Substitution in den beiden Orthostellungen zum N-Atom kann einen günstigen Einfluss auf die gewünschten Ausbeuten haben, wobei in diesem Fall $R^2$ vorzugsweise nicht Aryl bedeutet. Bevorzugt sind beide Orthostellungen substituiert, insbesondere mit $C_1$-$C_{12}$-Alkyl.

Geeignete Substituenten für $R^1$ sowie $R^2$ und $R^3$ in der Bedeutung von Aryl und Aralkyl sind:

$C_1$-$C_{12}$-, bevorzugt $C_1$-$C_6$- und besonders $C_1$-$C_4$-Alkyl, -Alkoxy oder -Alkylthio, z.B. Methyl, Ethyl, Propyl, n-, i- und t-Butyl, die Isomeren von Pentyl, Hexyl, Octyl, Nonyl, Decyl, Undecyl und Dodecyl, sowie entsprechende Alkoxy- und Alkylthioreste;

$C_1$-$C_6$-, vorzugsweise $C_1$-$C_4$-Haloalkyl mit vorzugsweise F und Cl als Halogen, z.B. Trifluor- oder Trichlormethyl, Difluorchlormethyl, Fluordichlormethyl, 1,1-Difluoreth-1-yl, 1,1-Dichloreth-1-yl, 1,1,1-Trichlor- oder -Trifluoreth-2-yl, Pentachlorethyl, Pentafluorethyl, 1,1,1-Trifluor-2,2-dichlorethyl, n-Perfluorpropyl, i-Perfluorpropyl, n-Perfluorbutyl, Fluor- oder Chlormethyl, Difluor- oder Dichlormethyl, 1-Fluor- oder -Chlor-eth-2-yl oder -eth-1-yl, 1-, 2-oder 3-Fluor- oder -Chlor-prop-1-yl oder -prop-2-yl oder -prop-3-yl, 1-Fluor- oder -Chlor-but-1-yl, -but-2-yl, -but-3-yl oder -but-4-yl, 2,3-Dichlor-prop-1-yl, 1-Chlor-2-fluor-prop-3-yl, 2,3-Dichlor-but-1-yl;

Halogen, bevorzugt F und Cl;

$C_6$-$C_{12}$-Aryl, -Aryloxy oder -Arylthio, in denen Aryl bevorzugt für Naphthyl und besonders Phenyl steht,

$C_7$-$C_{16}$-Aralkyl, -Aralkoxy und -Aralkylthio, in denen der Arylrest bevorzugt Naphthyl und besonders Phenyl ist und der Alkylenrest linear oder verzweigt ist und 1 bis 10, vorzugsweise 1 bis 6 und insbesondere 1-3 C-Atome enthält, z.B. Benzyl, Naphthylmethyl, 1-oder 2-Phenyleth-1-yl oder -eth-2-yl, 1-, 2- oder 3-Phenylprop-1-yl, -prop-2-yl oder -prop-3-yl, besonders bevorzugt ist Benzyl; die zuvor genannten Arylgruppen enthaltende Reste können ihrerseits ein- oder mehrfach substituiert sein, z.B. durch $C_1$-$C_4$-Alkyl, -Alkoxy oder -Alkylthio, Halogen, -OH, -CN, $-CONR^4R^5$ oder $-COOR^4$, wobei $R^4$ und $R^5$ die angegebenen Bedeutungen haben; Beispiele sind Methyl, Ethyl, n- und i-Propyl, Butyl, entsprechende Alkoxy- und Alkylthioreste, F, Cl, Br, Dimethyl-, Methylethyl-, Diethylcarbamoyl, Methoxy-, Ethoxy-, Phenoxy- und Benzyloxycarbonyl,

Sekundäramino mit 2 bis 24, vorzugsweise 2 bis 12 und besonders 2 bis 6 C-Atomen, wobei das Sekundäramino bevorzugt 2 Alkylgruppen enthält, z.B. Dimethyl-, Methylethyl-, Diethyl-, Methylpropyl-Methyl-n-butyl-, Di-n-Propyl-, Di-n-Butyl-, Di-n-Hexylamino;

$-CONR^4R^5$, worin $R^4$ und $R^5$ unabhängig voneinander $C_1$-$C_{12}$-, vorzugsweise $C_1$-$C_6$- und insbesondere $C_1$-$C_4$-Alkyl oder $R^4$ und $R^5$ zusammen Tetra- oder Pentamethylen oder 3-Oxapentylen sind, wobei das Alkyl linear oder verzweigt sein kann, z.B. Dimethyl-, Methylethyl-, Diethyl-, Methyl-n-propyl-, Ethyl-n-propyl-, Di-n-propyl-, Methyl-n-butyl-, Ethyl-n-butyl-, n-Propyl-n-butyl- und Di-n-butylcarbamoyl;

$-COOR^4$, worin $R^4$ $C_1$-$C_{12}$-, bevorzugt $C_1$-$C_6$-Alkyl ist, das linear oder verzweigt sein kann, z.B. Methyl, Ethyl, n- und i-Propyl, n-, i- und t-Butyl, und die Isomeren von Pentyl, Hexyl, Heptyl, Octyl, Nonyl, Decyl, Undecyl und Dodecyl.

$R^1$ als Aryl ist bevorzugt unsubstituiertes oder substituiertes Naphthyl und besonders Phenyl. $R^1$ als Heteroaryl ist bevorzugt ein 5- oder 6-gliedriger Ring mit 1 oder 2 gleichen oder verschiedenen Heteroatomen, besonders O, S oder N, das bevorzugt 4 oder 5 C-Atome enthält und das mit Benzol kondensiert sein kann. Beispiele für Heteroaromaten, von denen sich $R^1$ ableiten kann, sind Furan, Pyrrol, Thiophen, Pyridin,

Pyrimidin, Indol und Chinolin.

Für die Substituenten von $R^2$ und $R^3$ gelten die gleichen Bevorzugungen wie für die Substituenten von $R^1$.

$R^2$ und $R^3$ als Alkyl sind bevorzugt unsubstituiertes oder substituiertes $C_1$-$C_6$-, besonders $C_1$-$C_4$-Alkyl, das linear oder verzweigt sein kann. Beispiele sind Methyl, Ethyl, i- und n-Propyl, i-, n- und t-Butyl, die Isomeren von Pentyl, Hexyl, Heptyl, Octyl, Nonyl, Decyl, Undecyl und Dodecyl.

$R^2$ und $R^3$ als unsubstituiertes oder substituiertes Cycloalkyl enthalten bevorzugt 3 bis 6, besonders 5 oder 6 Ring-C-Atome. Beispiele sind Cyclopropyl, Cyclobutyl, Cyclopentyl, Cyclohexyl, Cycloheptyl und Cyclooctyl.

$R^2$ und $R^3$ als Aryl sind bevorzugt unsubstituiertes oder substituiertes Naphthyl und besonders Phenyl. $R^2$ und $R^3$ als Aralkyl sind bevorzugt unsubstituiertes oder substituiertes Phenylalkyl mit 1-10, bevorzugt 1 bis 6 und besonders 1 bis 4 C-Atomen im Alkylen, wobei das Alkylen linear oder verzweigt sein kann. Beispiele sind besonders Benzyl, sowie 1-Phenyleth-1-yl, 2-Phenyleth-1-yl, 1-Phenylprop-1-yl, 1-Phenylprop-2-yl, 1-Phenylprop-3-yl, 2-Phenylprop-1-yl, 2-Phenylprop-2-yl und 1 -Phenylbut-4-yl.

In $R^2$ und $R^3$ als -$CONR^4$ $R^5$ und -$COOR^4$ stellen $R^4$ und $R^5$ bevorzugt $C_1$-$C_6$-, besonders $C_1$-$C_4$-Alkyl oder $R^4$ und $R^5$ zusammen Tetramethylen, Pentamethylen oder 3-Oxapentylen dar. Beispiele für Alkyl sind zuvor erwähnt worden.

$R^2$ und $R^3$ zusammen als Alkylen sind bevorzugt durch 1 -O-, -S- oder -$NR^4$-, vorzugsweise -O-, unterbrochen. $R^2$ und $R^3$ zusammen bilden mit dem C-Atom bzw. mit der -N=C-Gruppe, an das sie gebunden sind, bevorzugt einen 5-oder 6-gliedrigen Ring. Für die Substituenten gelten die zuvor erwähnten Bevorzugungen. Als kondensiertes Alkylen sind $R^2$ und $R^3$ zusammen bevorzugt mit Benzol oder Pyridin kondensiertes Alkylen. Beispiele für Alkylen sind: Ethylen, 1,2-oder 1,3-Propylen, 1,2-, 1,3- oder 1,4-Butylen, 1,5-Pentylen und 1,6-Hexylen. Beispiele für unterbrochenes oder durch =O substituiertes Alkylen sind 2-Oxa-1, 3-propylen, 2-Oxa-1, 4-butylen, 2-Oxa-oder 3-Oxa-1,5-pentylen, 3-Thia-1,5-pentylen, 2-Thia-1,4-butylen, 2-Thia-1,3-propylen, 2-Methylimino-1,3-propylen, 2-Ethylimino-1,4-butylen, 2- oder 3-Methylimino-1,5-pentylen, 1-Oxo-2-oxa-1,3-propylen, 1-Oxo-2-oxa-1,4-butylen, 2-Oxo-3-oxa-1,4-butylen, 1-Oxa-2-oxo-1,5-pentylen. Beispiele für kondensiertes Alkylen sind:

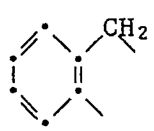 , 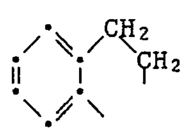 , 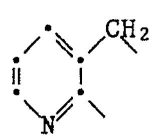 und 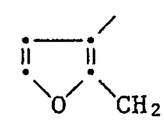 .

Beispiele für kondensiertes und unterbrochenes und gegebenenfalls mit =O substituiertes Alkylen sind

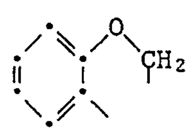 , 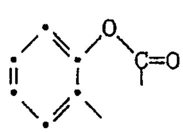 und 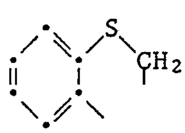 .

In einer bevorzugten Gruppe steht in Formel II $R^1$ für 2,6-Dimethylphen-1-yl oder 2-Methyl-6-ethylphen-1-yl, $R^2$ für Methyl und $R^3$ für Methoxymethyl.

N-Arylimine der Formel II sind bekannt oder sie können nach bekannten Verfahren aus Ketonen und Arylaminen hergestellt werden. In einer Ausführungsform des Verfahrens können die N-Arylimine der Formel II auch in situ aus den entsprechenden Ketonen und Arylaminen hergestellt werden.

Das Verfahren wird bevorzugt bei einer Temperatur von -20 bis 50 °C, besonders -20 bis 20 °C, insbesondere -20 bis 10 °C, und bevorzugt bei einem Wasserstoffdruck von $2 \cdot 10^5$ bis $3 \cdot 10^6$ Pa, besonders $8 \cdot 10^5$ bis $3 \cdot 10^6$ Pa durchgeführt.

In den Formeln III und IIIa kann X als Olefinligand z.B. Buten, Propen und besonders Ethylen sein und der Dienligand ist bevorzugt ein offenkettiges oder cyclisches Dien, dessen Diengruppen durch ein oder zwei C-Atome verknüpft sind. Das Dien ist bevorzugt Hexadien, Cyclooctadien oder Norbornadien.

Im chiralen Diphosphin sind die Phosphingruppen bevorzugt über eine aliphatische Gruppe mit 2-4 C-Atomen verknüpft, die durch $C_1$-$C_4$-Alkyl, $C_5$- oder $C_6$-Cycloalkyl, Phenyl oder Benzyl substituiert sein kann. Bei der aliphatischen Gruppe kann es sich um Alkylen oder um eine cycloaliphatische Gruppe mit 5 oder 6 Ring-C-Atomen oder um eine aliphatisch-heterocyclische Gruppe mit 1 bis 2 -O- oder =N-$C_1$-$C_{12}$-Alkyl,

oder -Acyl oder -Aminocarbonyl, -Phenyl oder -Benzyl und 3-5 C-Atomen im Ring handeln. Die Ringe können durch $C_1$-$C_4$-Alkyl, $C_5$-$C_7$-Cycloalkyl, Phenyl oder Benzyl substituiert sein.

Y in den Formeln III oder IIIa ist bevorzugt ein chirales Diphosphin, dessen Phosphingruppen durch 4 C-Atome verknüpft sind und das zusammen mit dem Ir-Atom einen 7-Ring bildet.

Die Phosphingruppen und Phosphinitgruppen enthalten bevorzugt $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe. Besonders bevorzugt sind t-Butyl, Phenyl, Benzyl oder Cyclohexyl. Geeignete chirale Diphosphine sind beschrieben in H.B. Kagan, Chiral Ligands for Asymmetric Catalysis, Asymmetric Synthesis, Volume 5, S. 13-23, Academic Press, Inc., N.Y. (1985).

Beispiele sind (Ph steht für Phenyl):

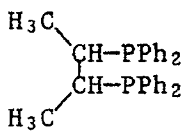 , 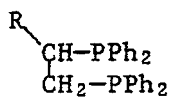 R = Methyl, Phenyl, Cyclohexyl,

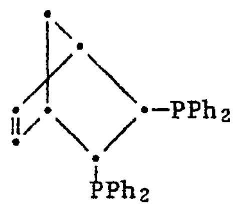 , 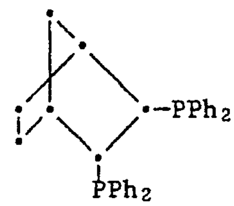 ,

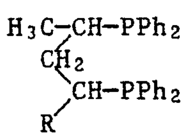 R=H,$CH_3$ , 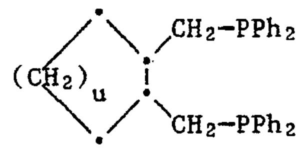 u=0, 1, 2 ,

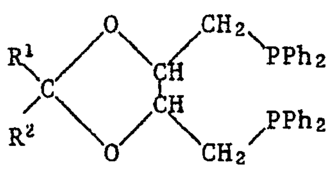 $R^1$=H,$CH_3$,Ph, $R^2$=H,$CH_3$,Ph 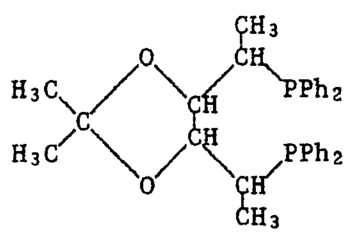 ,

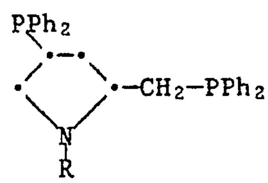 R=$-CO_2$-t-Butyl, -CO-t-Butyl, -CONHC$_1$-C$_4$-Alkyl,

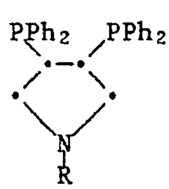 R=Benzyl, $C_1$-$C_4$-Alkyl.

Ein Beispiel für Disphosphinite ist 1-0-Phenyl-4,6-0-(R)-benzyliden-2,3-0-bis(diphenylphosphino)-$\beta$-D-glucopyranosid der Formel

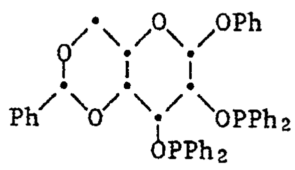

In Formel III steht Z bevorzugt für Cl oder Br. $A^{\ominus}$ in Formel IIIa steht bevorugt für $ClO_4^{\ominus}$, $CF_3SO_3^{\ominus}$, $BF_4^{\ominus}$, B-(Phenyl)$_4^{\ominus}$, $PF_6^{\ominus}$, $SbCl_6^{\ominus}$, $AsF_6^{\ominus}$ oder $SbF_6^{\ominus}$.

Eine bevorzugte Gruppe von Iridiumverbindungen sind solche der Formel III, worin X für Cyclooctadien, Z für Cl und Y für (R)- oder (S)-

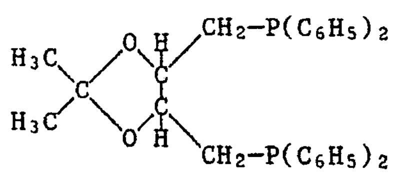

stehen.

Die Iridiumverbindungen der Formeln III und IIIa sind bekannt oder können nach bekannten Verfahren hergestellt werden, siehe z.B. R. Uson et al., Inorg. Chim. Acta 73, S. 275 ff (1983); S. Brunie et al., Journal of Organometallic Chemistry, 114 (1976), S. 225-235 und M. Green et al., J. Chem. Soc. (A), S. 2334 ff (1971).

Die Iridiumverbindungen können als isolierte Verbindungen eingesetzt werden. Es ist zweckmässig, die Verbindungen in situ herzustellen und direkt zu verwenden.

Die Iridiumverbindungen werden bevorzugt in Mengen von 0,01 bis 5, besonders 0,05 bis 2 Mol-% eingesetzt, bezogen auf die Verbindungen der Formel II.

Eine bevorzugte Verfahrensdurchführung ist dadurch gekennzeichnet, dass man zusätzlich ein Ammonium- oder Alkalimetallchlorid, -bromid oder -jodid zusetzt. Der Zusatz von Chloriden, Bromiden oder Jodiden ist besonders dann zweckmässig, wenn man Verbindungen der Formel IIIa als Katalysatoren einsetzt. Die Chloride, Bromide und Jodide werden bevorzugt in Mengen von 0,01 bis 100, besonders 0,05 bis 50 Mol-% eingesetzt, bezogen auf die Verbindungen der Formel II. Als Salze sind die Jodide bevorzugt. Ammonium ist bevorzugt Tetraalkylammonium mit 1 bis 6 C-Atomen in den Alkylgruppen und als Alkalimetall ist Natrium, Lithium oder Kalium bevorzugt.

Die Reaktion kann ohne oder in Gegenwart von Lösungsmitteln durchgeführt werden. Geeignete Lösungsmittel, die alleine oder als Mischung von Lösungsmitteln eingesetzt werden können, sind zum Beispiel:

Aliphatische und aromatische Kohlenwasserstoffe, wie z.B. Pentan, Hexan, Cyclohexan, Methylcyclohexan, Benzol, Toluol und Xylol; Alkohole, wie z.B. Methanol, Ethanol, Propanol und Butanol; Ether, wie z.B. Diethylether, Diethylenglykoldimethylether, Tetrahydrofuran und Dioxan; Halogenkohlenwasserstoffe, wie z.B. Methylenchlorid, Chloroform, 1,1,2,2-Tetrachlorethan und Chlorbenzol; Ester und Lactone, wie z.B. Essigsäureethylester, Butyrolacton und Valerolacton; Säureamide und Lactame, wie z.B. Dimethylformamid, Dimethylacetamid und N-Methylpyrrolidon.

Die Verbindungen der Formel I sind biologisch aktive Substanzen oder Zwischenprodukte zur Herstellung solcher Substanzen mit einer N-Arylsekundäramingruppe, insbesondere im Bereich der Pharmazeutika und Agrochemikalien. So wirken z.B. o,o-Dialkylarylketaminderivate, insbesondere solche mit Alkyl- und/oder Alkoxyalkylgruppen, als Fungizide, besonders als Herbizide. Bei den Derivaten kann es sich um Aminsalze, Säureamide, z.B. von Chloressigsäure, tertiäre Amine und Ammoniumsalze handeln (siehe z.B. EP-A-0 077 755 und EP-A-01 15 470).

Die nachfolgenden Beispiele erläutern die Erfindung näher.

Beispiele 1-10:

In einen 250 ml Zweihalskolben werden unter $N_2$-Schutzgas 160 mmol N(2,6-Dimethylphen-1-yl)-methoxymethyl-methylketimin eingetragen. Der Kolben wird auf $5 \cdot 10^3$ Pa evakuiert und mit Stickstoff

gespült. Danach werden je 30 ml Methanol und Benzol zugegeben und 2 Minuten bei Raumtemperatur gerührt (Lösung A).

In einen 50 ml Zweihalskolben werden unter $N_2$-Schutzgas je 10 ml Methanol und Benzol eingetragen. Danach werden nacheinander $4 \cdot 10^{-5}$ mol [Ir(Cyclooctadien-)Cl]$_2$, $8{,}8 \cdot 10^{-5}$ mol Diphosphin und $1{,}2 \cdot 10^{-3}$ mol Tetrabutylammoniumjodid (nur bei Beispiel 1 und 2) zugefügt. Nach jeder Zugabe wird so lange gerührt, bis eine homogene Lösung vorliegt (Lösung B).

In einen 0,3 l Stahlautoklaven werden unter Luftausschluss mit einer Kapillare die Lösungen A und B nacheinander eingetragen. Durch ein Gaseinlassventil werden $2 \cdot 10^6$ Pa Wasserstoff aufgepresst. Gleichzeitig werden in ein 100 ml Reservoir $1{,}4 \cdot 10^7$ Pa Wasserstoff aufgepresst. Die Temperatur beträgt 20-22 °C. Die Reaktion wird solange bei einem konstanten Wasserstoffdruck von $2 \cdot 10^6$ Pa durchgeführt, bis keine Wasserstoffaufnahme mehr erfolgt. Danach wird das Reaktionsgemisch mit Stickstoff in einen 250 ml Kolben gespült. Das Lösungsmittel wird bei 80 °C am Rotationsverdampfer entfernt. Man erhält ein Rohprodukt, das man im Hochvakuum (1-10 Pa) destilliert. Anschliessend bestimmt man polarometrisch die optische Ausbeute (A.F. Lee et al., J. Chem. Arc. 1954, 145), oder mittels $^1$H-HMR unter Verwendung von Shiftreagenzien.

Im Beispiel 2 werden 80 mMol Ketimin verwendet. In den übrigen Beispielen beträgt die Katalysatorkonzentration 1 Mol-% (40 mMol), wobei 20 ml Lösungsmittel verwendet werden. Die Reaktionen werden in einem 120 ml Glas- oder 65 ml Stahlautoklaven durchgeführt.

Die optischen Ausbeuten (ee in %) Reaktionszeiten, Umsatz, Lösungsmittel und Reaktionstemperaturen sind in Tabelle 1 angegeben.

Tabelle 1:

| Beispiel | Diphosphin | Tempe-ratur (°C) | Lösungsmittel | Reaktions-zeit (Stunden) | Umsatz (%) | Optische Ausbeute % ee (Kon-figuration) |
|---|---|---|---|---|---|---|
| 1 | 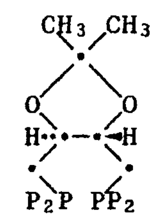 (+)-DIOP | 20 | Methanol Benzol (1:1) | 100 | 96 | 68(S) |
| 2 | (+)-DIOP | 20 | Methanol Benzol (1:1) | 48 | 72 | 60.5(S) |
| 3 | (+)-DIOP | 20 | Methanol/ Benzol (1:1) | 18 | 93 | 38(S) |
| 4 | 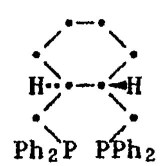 C6-DIOP | 25 | Methanol/ Benzol (1:1) | 43 | 30 | 28(R) |
| 5 | 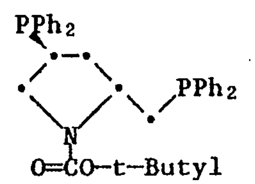 BPPM | 20 | Methanol/ Benzol (1:1) | 22 | 93 | 56(S) |

Tabelle 1: (Fortsetzung)

| Beispiel | Diphosphin | Temperatur (°C) | Lösungsmittel | Reaktionszeit (Stunden) | Umsatz (%) | Optische Ausbeute % ee (Konfiguration) |
|---|---|---|---|---|---|---|
| 6 | BPPM | 20 | Tetrahydrofuran | 22 | 99 | 73(S) |
| 7 | 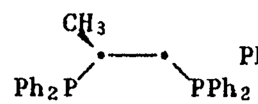 PROPHOS | 50 | Methanol/ Benzol (1:1) | 65 | 53 | 19(S) |
| 8 | 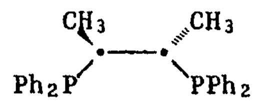 CHIRAPHOS | 50 | Methanol/ Benzol (1:1) | 24 | 54 | 37(S) |
| 9 | 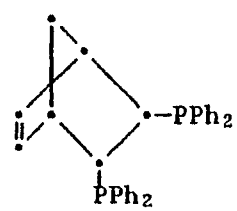 (+)-NORPHOS | 18 | $CH_2Cl$ | 65 | 99 | 54(R) |
| 10 | 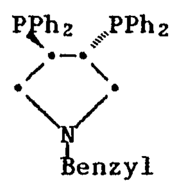 DEGUPHOS | 50 | Methanol/ Benzol (1:1) | 20 | 77 | 33(R) |

Beispiele 11-14:

Es wird wie in den Beispielen 3-10 verfahren und die Reaktionsbedingungen verändert. Als Ketimin wird N(2-Methyl-6-ethylphen-1-yl)-methyl-methoxymethyl-ketimin verwendet. Als Diphosphin wird (-)-DIOP (Beispiel 11) und (+)-DIOP (Beispiele 12-14) eingesetzt. Der Wasserstoffdruck beträgt $2 \cdot 10^6$ Pa. Die

Ergebnisse sind in Tabelle 2 dargestellt.

Tabelle 2:

| Beispiel | Zusatz (Aequivalente pro Iridium) | Temperatur (°C) | Lösungsmittel | Reaktionszeit (Stunden) | Umsatz (%) | Optische Ausbeute % ee (Konfiguration) |
|---|---|---|---|---|---|---|
| 11 | KI (1) | 50 | Methanol/Benzol | 22,5 | 90 | 49(R) |
| 12[1)] | Tetrabutylammoniumjodid (2) | 18 | Methanol/Benzol | 20 | 91 | 58(S) |
| 13 | Tetrabutylammoniumjodid (2) | 18 | Tetrahydrofuran | 20,5 | 98 | 61(S) |
| 14 | Tetrabutylammoniumjodid (2) | 18 | Ethanol | 20 | 97 | 53(S) |

1) 80 mMol Ketimin

Beispiele 15-23:

Es wird wie in den Beispielen 3-10 verfahren unter Verwendung verschiedener Ketimine. In Beispiel 23 wird Methylenchlorid als Lösungsmittel, sonst Benzol/Methanol verwendet. Die Ergebnisse sind in Tabelle 3 dargestellt.

Tabelle 3:

| Beispiel | Ketimin 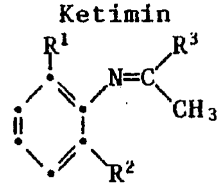 | Diphosphin | Zusatz* (Aequivalente pro Iridium) | Reaktionszeit (Stunden) | Umsatz (%) | Optische Ausbeute % ee (Konfiguration) |
|---|---|---|---|---|---|---|
| 15 | $R^1=R^2=H$ $R^3=n-C_3H_7$ | (-)-DIOP | - | 19 | 99 | 6 |
| 16 | $R^1=R^2=H$ $R^3$=Phenyl | (+)-DIOP | 2 | 18 | 90 | 22 |
| 17 | $R^1=R^2=H$ $R^3$=Phenyl | BPPM | 2 | 17 | 65 | 9.5 |
| 18 | $R^1=R^2=H$ $R^3$=Benzyl | (+)-DIOP | 2 | 19,5 | 99 | 30 |
| 19 | $R^1=R^2=CH_3$ $R^3=n-C_3H_7$ | (+)-DIOP | 2 | 17 | 65 | 52 |

*) Tetrabutylammoniumjodid (TBAI)

Tabelle 3: (Fortsetzung)

| Bei-spiel | Ketimin | Diphosphin | Zusatz* (Aequi-valente pro Iridium) | Reaktions-zeit (Stun-den) | Umsatz (%) | Optische Ausbeute % ee (Kon-figuration) |
|---|---|---|---|---|---|---|
| | 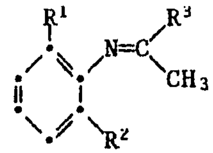 | | | | | |
| 20 | $R^1=R^2=CH_3$ $R^3=n-C_3H_7$ | DEGUPHOS | 2 | 42 | 62 | 1 |
| 21 | 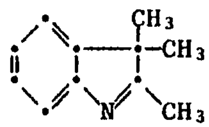 | (+)-DIOP | 2 | 7 | 99 | 66 |
| 22 | 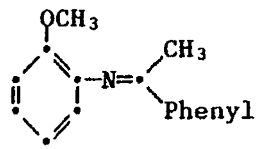 | (+)-DIOP | 2 | 20 | 99 | 30 |
| 23 | 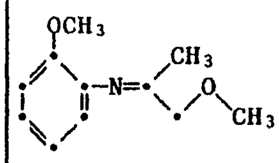 | (+)-DIOP | 2 | 6 | 99 | 7 |
| 23a | 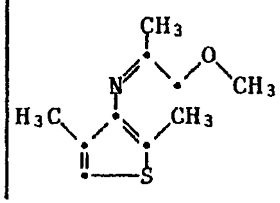 | (+)-DIOP | 2 | 4 | 97 | 57 |

*Tetrabutylammoniumiodid (TBAI)

**Beispiele 24-40:**

Es wird wie in den Beispielen 3-10 verfahren. Die Reaktionsbedingungen und Ergebnisse sind in Tabelle 4 dargestellt.

**Tabelle 4:**

| Beispiel | Diphosphin | Zusatz (Aequivalente pro Iridium) | Druck ($10^6$ Pa) | Temperatur (°C) | Lösungsmittel | Reaktionszeit (Stunden) | Umsatz (%) | Optische Ausbeute % ee (Konfiguration) |
|---|---|---|---|---|---|---|---|---|
| 24 | (+)-DIOP | KJ (1) | 2 | 18 | Methanol/Benzol | 66 | 98 | 62,5(S) |
| 25 | (+)-DIOP | KJ (2) | 2 | 18 | Methanol/Benzol | 16 | 98 | 63(S) |
| 26 | (+)-DIOP | KJ (5) | 2 | 18 | Methanol/Benzol | 18 | 96 | 62,5(S) |
| 27 | (+)-DIOP | KJ (10) | 2 | 18 | Methanol/Benzol | 38 | 100 | 65(S) |
| 28 | (+)-DIOP | KJ (20) | 2 | 18 | Methanol/Benzol | 42,5 | 88 | 60,5(S) |
| 29 | (+)-DIOP | - | 2 | 18 | Ethanol | 19 | 97 | 55(S) |
| 30 | (+)-DIOP | - | 2 | 18 | Benzol | 23 | 88 | 65(S) |
| 31 | (+)-DIOP | - | 2 | 18 | Tetrahydrofuran | 18 | 97 | 68,5(S) |
| 32 | (+)-DIOP | - | 2 | 18 | $CH_2Cl_2$ | 18 | 98 | 44(S) |
| 33 | (+)-DIOP | - | 1 | 18 | Methanol/Benzol | 5 | 42 | 57(S) |
| 34 | (+)-DIOP | - | 1 | 18 | Methanol/Benzol | 20 | 89 | 57(S) |

Tabelle 4: (Fortsetzung)

| Beispiel | Diphosphin | Zusatz (Aequivalente pro Iridium) | Druck ($10^6$ Pa) | Temperatur (°C) | Lösungsmittel | Reaktionszeit (Stunden) | Umsatz (%) | Optische Ausbeute % ee (Konfiguration) |
|---|---|---|---|---|---|---|---|---|
| 35 | (+)-DIOP | - | 1 | 25 | Methanol/Benzol | 42 | 94 | 52(S) |
| 36 | (+)-DIOP | - | 1 | 50 | Methanol/Benzol | 22 | 67 | 47(S) |
| 37 | (+)-NORPHOS | - | 2 | 50 | Methanol/Benzol | 66 | 60 | 27(R) |
| 38 | (+)-NORPHOS | - | 2 | 18 | Methanol/Benzol | 41 | 77 | 52,5(R) |
| 39 | DEGUPHOS | - | 2 | 18 | $CH_2Cl_2$ | 19 | 98 | 41,5(R) |
| 40 | (+)-DIOP | - | 2 | 25 | Methanol/Benzol | 17 | 96 | 39,5(S) |

Beispiel 41:

Es wird wie in den Beispielen 3-10 verfahren, jedoch unter Verwendung von 1-0-Phenyl-4,6-0-(R)-benzyliden-2,3-0-bis(diphenylphosphino)-β-D-glucopyranosid, 2 Aequivalenten TBAI pro Iridium und N-(2-Methyl-6-ethylphen-1-yl)-methyl-methoxmethylketimin. Die Reaktionszeit beträgt 21,5 Stunden. Bei einem Umsatz von 67% beträgt der Enantiomerenüberschuss (ee) 44% (S).

Beispiel 42:

Es wird gemäss den Reaktionsbedingungen von Beispiel 3 verfahren und das Ketimin aus 40 mMol 2,6-Dimethylanilin und 40 mMol Methoxyaceton in situ erzeugt. Als Lösungsmittel wird THF verwendet. Die Reaktionszeit ist 144 Stunden. Bei einem Umsatz von 37% wird ein Enantiomerenüberschuss von 64% ee (S) erzielt.

## Patentansprüche

1. Verfahren zur Herstellung von optisch aktiven sekundären N-Arylaminen der Formel I

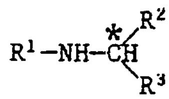 (I),

worin $R^1$ $C_6$-$C_{12}$-Aryl oder über ein Ring-C-Atom gebundenes $C_4$-$C_{11}$-Heteroaryl mit 1 oder 2 Heteroatomen im Ring bedeutet, die durch $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CN, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, -CONR$^4$R$^5$ oder -COOR$^4$ substituiert sein können, Sekundäramino mit 2 bis 24 C-Atomen,

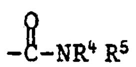 oder 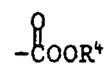 ,

wobei $R^4$ und $R^5$ unabhängig voneinander $C_1$-$C_{12}$-Alkyl, Phenyl oder Benzyl oder $R^4$ und $R^5$ zusammen Tetra- oder Pentamethylen oder 3-Oxapentylen sind, substituiert sein können; $R^2$ und $R^3$ voneinander verschieden sind und gegebenenfalls durch -OH, -CN, Halogen, $C_1$-$C_{12}$-Alkoxy, Phenoxy, Benzyloxy, Sekundäramino mit 2 bis 24 C-Atomen,

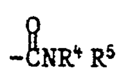

oder -COOR$^4$ substituiertes $C_1$-$C_{12}$-Alkyl oder Cycloalkyl mit 3-8 Ring-C-Atomen, gegebenenfalls wie $R^1$ substituiertes $C_6$-$C_{12}$-Aryl oder $C_7$-$C_{16}$-Aralkyl, -CONR$^4$R$^5$ oder -COOR$^4$ darstellen, worin $R^4$ und $R^5$ die zuvor angegebene Bedeutung haben; oder $R^1$ die zuvor angegbene Bedeutung hat und $R^2$ und $R^3$ zusammen gegebenenfalls durch 1 oder 2 -O-, -S- oder -NR$^4$- unterbrochenes, und/oder gegebenenfalls durch =0 oder wie zuvor für $R^2$ und $R^3$ in der Bedeutung von Alkyl sustituiertes, und/oder mit Benzol, Furan, Thiophen oder Pyrrol kondensiertes Alkylen mit 2 bis 5 C-Atomen sind, und * für überwiegend R- oder S-Konfiguration steht, durch asymmetrisch katalysierte Hydrierung von N-arylierten prochiralen Ketiminen der Formel II

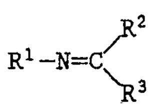 (II),

worin $R^1$, $R^2$ und $R^3$ die zuvor angegebenen Bedeutungen haben, in Gegenwart von Komplexsalzen eines Edelmetalls mit chiralen Liganden, dadurch gekennzeichnet, dass man die Hydrierung bei einer Temperatur von -20 bis 80°C und einem Wasserstoffdruck von $10^5$ Pa bis $6 \cdot 10^6$ Pa vornimmt und dem Reaktionsgemisch katalytische Mengen einer Iridiumverbindung der Formel III oder IIIa

[XIrYZ] (III) oder $[XIrY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Ir-Atom einen 5-, 6- oder 7-Ring bildet, oder Y ein chirales Diphosphinit, dessen Phosphinitgruppen über 2 C-Atome verknüpft sind und das zusammen mit dem Ir-Atom einen 7-Ring bildet, bedeutet, Z für Cl, Br oder J steht, und $A^{\ominus}$ das Anion einer Sauerstoff- oder Komplexsäure darstellt.

2. Verfahren gemäss Anspruch 1, dadurch gekennzeichnet, dass die Reaktionstemperatur -20 bis 50 °C beträgt.

3. Verfahren gemäss Anspruch 1, dadurch gekennzeichnet, dass der Wasserstoffdruck $2 \cdot 10^5$ Pa bis $3 \cdot 10^6$ Pa beträgt.

4. Verfahren gemäss Anspruch 1, dadurch gekennzeichnet, dass X in den Formeln III und IIIa zwei Ethylen oder ein offenkettiges oder cyclisches Dien ist, dessen Diengruppen über 1 oder 2 C-Atome verknüpft sind.

5. Verfahren gemäss Anspruch 4, dadurch gekennzeichnet, dass das Dien Hexadien, Norbornadien oder Cyclooctadien ist.

6. Verfahren gemäss Anspruch 1, dadurch gekennzeichnet, dass Y in den Formeln III und IIIa ein chirales Diphosphin ist, dessen Phosphingruppen durch 4 C-Atome verknüpft sind und das zusammen mit dem Ir-Atom einen 7-Ring bildet.

7. Verfahren gemäss Anspruch 1, dadurch gekennzeichnet, dass die Phosphingruppen $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe enthalten.

8. Verfahren gemäss Anspruch 1, dadurch gekennzeichnet,dass $A^{\ominus}$ für $ClO_4^{\ominus}$, $CF_3SO_3^{\ominus}$, $BF_4^{\ominus}$, B(Phenyl)-$_4^{\ominus}$, $PF_6^{\ominus}$, $SbCl_6^{\ominus}$, $AsF_6^{\ominus}$ oder $SbF_6^{\ominus}$ steht.

9. Verfahren gemäss Anspruch 1, dadurch gekennzeichnet, dass die Iridiumverbindung in einer Menge von 0,01 bis 5 Mol-% zugegeben wird, bezogen auf die Verbindung der Formel II.

10. Verfahren gemäss Anspruch 1, dadurch gekennzeichnet, dass man zusätzlich ein Ammonium- oder Alkalimetallchlorid, -bromid oder -jodid zugibt.

11. Verfahren gemäss Anspruch 1, dadurch gekennzeichnet, dass in Formel III X für Cyclooctadien, Z für Cl und Y für (R)- oder (S)-

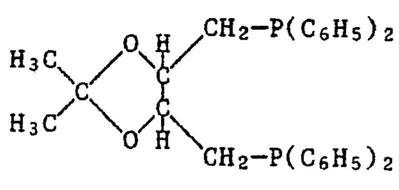

stehen

12. Verfahren gemäss Anspruch 1, dadurch gekennzeichnet, dass in Formel II $R^1$ für 2,6-Dimethylphen-1-yl oder 2-Methyl-6-ethylphen-1-yl, $R^2$ für Methyl und $R^3$ für Methoxymethyl stehen.

13. Verfahren gemäss Anspruch 1, dadurch gekennzeichnet, dass es sich bei dem Diphosphonit um 1-O-Phenyl-4,6-O-(R)-benzyliden-2,3-O-bis(diphenylphosphino)-$\beta$-D-glycopyranosid handelt.

## Claims

**1.** A process for the preparation of an optically active secondary N-arylamine of the formula I

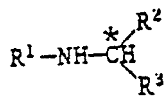

(I)

in which $R^1$ is $C_6$-$C_{12}$aryl or $C_4$-$C_{11}$heteroaryl which has 1 or 2 heteroatoms in the ring and is bonded via a ring C atom, it being possible for these radicals to be substituted by $C_1$-$C_{12}$alkyl, $C_1$-$C_{12}$alkoxy, $C_1$-$C_{12}$alkylthio, $C_1$-$C_6$haloalkyl, halogen, -OH, -CN, $C_6$-$C_{12}$aryl or -aryloxy or -arylthio, $C_7$-$C_{16}$aralkyl or -aralkoxy or aralkylthio, it being possible for the aryl radicals in turn to be substituted by $C_1$-$C_4$alkyl, -alkoxy, -alkylthio, halogen, -OH, -CN, $-CONR^4R^5$ or $-COOR^4$,
secondary amino with 2 to 24 C atoms,

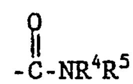

or $-COOR^4$, in which $R^4$ and $R^5$ independently of one another are $C_1$-$C_{12}$alkyl, phenyl or benzyl or $R^4$ and $R^5$ together are tetra- or pentamethylene or 3-oxapentylene; $R^2$ and $R^3$ differ from one another and are $C_1$-$C_{12}$alkyl or cycloalkyl with 3-8 ring C atoms, which are unsubstituted or substituted by -OH, -CN, halogen, $C_1$-$C_{12}$alkoxy, phenoxy, benzyloxy, secondary amino with 2 to 24 C atoms,

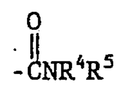

or $-COOR^4$, $C_6$-$C_{12}$aryl or $C_7$-$C_{16}$aralkyl which is unsubstituted or substituted in the same way as $R^1$, $-CONR^4R^5$ or $-COOR^4$, in which $R^4$ and $R^5$ are as defined above; or $R^1$ is as defined above and $R^2$ and $R^3$ together are alkylene which has 2 to 5 C atoms and is uninterrupted or interrupted by 1 or 2 -O-, -S- or $-NR^4$- and/or is unsubstituted or substituted by =O or as described above for $R^2$ and $R^3$ alkyl, and/or is fused with benzene, furan, thiophene or pyrrole, and * is chiefly the R- or S- configuration, by asymmetrically catalyzed hydrogenation of an N-arylated prochiral ketimine

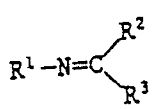

(II)

in which $R^1$, $R^2$ and $R^3$ are as defined above, in the presence of a complex salt of a noble metal with chiral ligands, which comprises carrying out the hydrogenation at a temperature of -20 to 80° C under a hydrogen pressure of $10^5$ Pa to $6.10^6$ Pa and adding to the reaction mixture catalytic amounts of an iridium compound of the formula III or IIIa

[XIrYZ] (III) or $[XIrY]^{\oplus}A^{\ominus}$ (111a)

in which X is two olefin ligands or one diene ligand, Y is a chiral diphosphine, the secondary phosphine groups of which are linked by 2-4 C atoms and which, together with the Ir atom, forms a 5-, 6- or 7-ring, or Y is a chiral diphosphinite, the phosphinite groups of which are linked via 2 C atoms and which together with the Ir atom forms a 7-ring, Z is Cl, Br or I and $A^{\ominus}$ is the anion of an oxygen acid or complex acid.

**2.** The process according to claim 1, wherein the reaction temperature is -20 to 50° C.

3. The process according to claim 1, wherein the hydrogen pressure is $2.10^5$ Pa to $3.10^6$ Pa.

4. The process according to claim 1, wherein X in the formulae III and IIIa is two ethylene or one open-chain or cyclic diene, the diene groups of which are linked via 1 or 2 C atoms.

5. The process according to claim 4, wherein the diene is hexadiene, norbornadiene or cyclooctadiene.

6. The process according to claim 1, wherein Y in the formulae III and IIIa is a chiral diphosphine, the phosphine groups of which are linked by 4 C atoms and which together with the Ir atom forms a 7-ring.

7. The process according to claim 1, wherein the phosphine groups contain $C_1$-$C_{12}$alkyl, cycloalkyl which has 5 to 8 ring C atoms and can be substituted by 1 to 3 $C_1$-$C_6$alkyl groups, phenyl, $C_7$-$C_{12}$phenylalkyl or alkylphenylalkyl with 1 to 6 C atoms in the alkyl groups and 1 to 5 C atoms in the alkylene group.

8. The process according to claim 1, wherein $A^\ominus$ is $C1O4^\ominus$, $CF_3SO_3^\ominus$, $BF_4^\ominus$ $B(phenyl)_4^\ominus$, $PF_6^\ominus$, $SbCl_6^\ominus$, $AsF_6^\ominus$, or $SbF_6^\ominus$.

9. The process according to claim 1, wherein the iridium compound is added in an amount of 0.01 to 5 mol %, based on the compound of the formula II.

10. The process according to claim 1, wherein an ammonium or alkali metal chloride, bromide or iodide is additionally added.

11. The process according to claim 1, wherein, in formula III, X is cyclooctadiene, Z is Cl and Y is (R)- or (S)-

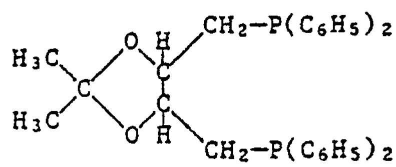

12. The process according to claim 1, wherein, in formula II, $R^1$ is 2,6-dimethylphen-1-yl or 2-methyl-6-ethylphen-1-yl, $R^2$ is methyl and $R^3$ is methoxymethyl.

13. The process according to claim 1, wherein the diphosphonite is 1-O-phenyl-4,6-O- (R)-benzylidene-2,3-0-bis(diphenylphosphino)-$\beta$-D-glycopyranoside.

## Revendications

1. Procédé de préparation de N-arylamines secon-daires, optiquement actives de formule I :

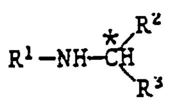 (I),

dans laquelle $R^1$ représente aryle en $C_6$-$C_{12}$ ou hétéroaryle en $C_4$-$C_{11}$ avec 1 ou 2 hétéroatomes dans le cycle, lié par l'intermédiaire d'un atome de C du cycle, qui peuvent être substitués par alkyle en $C_1$-$C_{12}$, alcoxy en $C_1$-$C_{12}$, alkythio en $C_1$-$C_{12}$, halogénoalkyle en $C_1$-$C_6$, halogène, -OH, -CN, aryle ou aryloxy ou arylthio en $C_6$-$C_{12}$, aralkyle ou aralcoxy ou aralkylthio en $C_7$-$C_{16}$, les radicaux aryles pouvant eux-mêmes être substitués par alkyle, alcoxy, alkylthio en $C_1C_4$, halogène, -OH, -CN, $-CONR^4R^5$ ou $-COOR^4$ ; ou par un radical amino secondaire avec 2 à 24 atomes de C,

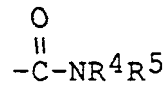

ou $-COOR^4$, $R^4$ et $R^5$ étant indépendamment l'un de l'autre, alkyle en $C_1$-$C_{12}$, phényle ou benzyle ou $R^4$ et $R^5$ étant ensemble tétra- ou pentaméthylène ou 3-oxapentylène;
$R^2$ et $R^3$ sont différents l'un de l'autre et représentent alkyle en $C_1$-$C_{12}$ ou cycloalkyle avec 3-8 atomes de C dans le cycle, éventuellement substitués par -OH, -CN, halogène, alcoxy en $C_1$-$C_{12}$, phénoxy, benzyloxy, un radical amino secondaire avec 2 à 24 atomes de C, $-CONR^4R^5$ ou $-COOR^4$ ou représentent aryle en $C_6$-$C_{12}$, ou aralkyle en $C_7$-$C_{16}$ éventuellement substitué comme $R^1$, $-CONR^4R^5$ ou $-COOR^4$, $R^4$ et $R^5$ ayant la signification indiquée plus haut ; ou $R^1$ a la signification donnée précédemment et $R^2$ et $R^3$ sont ensemble alkylène avec 2 à 5 atomes de C, éventuellement interrompu par 1 ou 2 -O-, -S- ou $-NR^4$-, et/ou éventuellement substitué par =O comme plus haut pour $R^2$ et $R^3$ dans la signification de alkyle, et/ou condensé avec un cycle benzène, furane, thiophène ou pyrrole,
et * remplace une configuration principalement R ou S, par hydrogénation catalytique asymétrique de cétimines prochirales, N-arylées, de formule II :

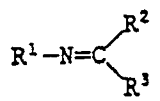 (II),

dans laquelle $R^1$, $R^2$ et $R^3$ ont les significations indiquées plus haut, en présence de sels complexes d'un métal précieux avec des ligands chiraux, lequel procédé est caractérisé en ce qu'on effectue l'hydrogénation à une température de -20 à 80 °C et sous une pression d'hydrogène de $10^5$ Pa à $6.10^6$ Pa et que l'on ajoute au mélange réactionnel des quantités catalytiques d'un composé de l'iridium de formule III ou IIIa :

$[XIrYZ]$ (III) ou $[XIrY]^{\oplus}A^{\ominus}$ (IIIa)

où X remplace deux ligands oléfiniques ou un ligand diénique, Y remplace une diphosphine chirale, dont les groupes phosphine secondaire sont reliés par 2-4 atomes de C et qui forme avec l'atome de Ir un cycle à 5, 6 ou 7 chaînons, ou Y représente un diphosphirtite chiral dont les groupes phosphinites sont reliés par l'intermédiaire de deux atomes de C et qui forme, ensemble avec l'atome de Ir, un cycle à 7 chaînons, Z remplace Cl, Br ou I, et $A^-$ représente l'anion d'un acide oxygéné ou complexe.

2. Procédé selon la revendication 1, caractérisé en ce que la température de réaction est de -20 à 50 °C.

3. Procédé selon la revendication 1, caractérisé en ce que la pression d'hydrogène est de $2.10^5$ Pa à $3.10^6$ Pa.

4. Procédé selon la revendication 1, caractérisé en ce que X dans les formules III et IIIa est deux éthylène ou un diène à chaîne ouverte ou cyclique, dont les groupes diène sont reliés par 1 ou 2 atomes de C.

5. Procédé selon la revendication 4, caractérisé en ce que le diène est l'hexadiène, le norbornadiène ou le cyclooctadiène.

6. Procédé selon la revendication 1, caractérisé en ce que Y dans les formules III et IIIa est une diphosphine chirale, dont les groupes phosphine sont reliés par 4 atomes de C et qui forme, ensemble avec l'atome de Ir un cycle à 7 chaînons.

7. Procédé selon la revendication 1, caractérisé en ce que les groupes phosphine contiennent alkyle en $C_1$-$C_{12}$, cycloalkyle avec 5 à 8 atomes de C dans le cycle, qui peut être substitué par 1 à 3 groupes alkyle en $C_1$-$C_6$ ; phényle, phénylalkyle en $C_7$-$C_{12}$ ou alkylphénylalkyle avec I à 6 atomes de C dans

les groupes alkyle et 1 à 5 atomes de C dans le groupe alkylène.

8. Procédé selon la revendication 1, caractérisé en ce que $A^-$ remplace $C1O_4^-$, $CF_3SO_3^-$, $BF_4^-$, B-(phényle)$4^-$, $PF_6^-$, $SbCl_6^-$, $AsF_6^-$ ou $SbF_6^-$.

9. Procédé selon la revendication 1 caractérisé en ce que le composé d'iridium est ajouté en une quantité de 0,01 à 5 mol%, par rapport au composé de formule II

10. Procédé selon la revendication 1, caractérisé en ce qu'on ajoute en outre un chlorure, bromure ou iodure d'ammonium ou de métal alcalin.

11. Procédé selon la revendication 1, caractérisé en ce que dans la formule III, X remplace cyclooctadiène, Z remplace Cl et Y remplace (R)- ou (S)-

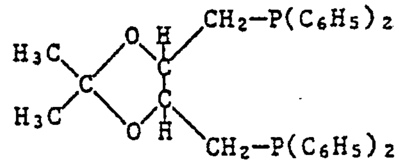

12. Procédé selon la revendication 1, caractérisé en ce que dans la formule II, $R^1$ remplace 2,6-diméthylphèn-1-yle ou 2-méthyl-6-éthylphèn-1-yle, $R^2$ remplace méthyle et $R^3$ remplace méthoxyméthyle.

13. Procédé selon la revendication 1, caractérisé en ce que le diphosphonite est le 1-O-phényl-4,6-O-(R)-benzylidène-2,3,0-bis(diphényl-phosphino)-$\beta$-D-glycopyrannoside.